# NOUVEAU FASCICULE DE BREVET EUROPEEN

(11) **EP 1 586 304 B2**
(45) Date de publication et mention de la décision concernant l'opposition: **17.08.2011**
(45) Mention de la délivrance du brevet: 18.04.2007
(21) Numéro de dépôt: 05290592.4
(22) Date de dépôt: 17.03.2005
(51) Int. Cl.: A61K 8/06, A61K 8/90, A61K 8/91, A61K 8/81, A61Q 15/00

(54) **Composition cosmétique déodorante du type émulsion eau-dans-huile contenant un dérivé polyoléfine comportant au moins une partie polaire comme emulsionnant**
Desodorierende kosmetische Zusammensetzung vom Typ Wasser-in -Öl die ein Polyolefinderivat mit mindestens einem polaren Teil als Emulgator enthält
Water in oil emulsion type deodorant cosmetic composition containing a polyolelfin derivative comprising at least one polar part as emulsifier

(30) Priorité: 15.04.2004 FR 0403941
(43) Date de publication de la demande: 19.10.2005
(73) Titulaire: L'Oréal, 75008 Paris (FR)
(72) Inventeur: Lemoine, Cyril, 95380 Puiseux-en-France (FR); Lebon-Hipolite, Emmanuelle, 60430 Abbecourt (FR)
(74) Mandataire: Miszputen, Laurent

(56) Documents cités:
- EP-A- 1 316 303
- EP-A2- 1 192 934
- WO-A1-93/14742
- FR-A- 2 811 565
- FR-A- 2 841 139

## Description

L'invention concerne une composition cosmétique comprenant dans un support du type émulsion eau-dans-huile :
(A) au moins un sel actif déodorant ;
(B) au moins un émulsionnant oligomère ou un polymère dérivé de polyoléfine, comportant au moins une partie polaire constituée de polyalkylène glycols, de polyalkylène imines, d'acides ou de diacides carboxyliques, de leurs anhydrides ou dérivés, et leurs mélanges et comportant une partie apolaire polyoléfinique comprenant au moins 40 atomes de carbone.

La présente invention a pour objet également un procédé cosmétique de traitement de la transpiration, consistant à appliquer sur la surface de la peau une quantité efficace d'une telle composition.

Dans le domaine cosmétique, il est bien connu d'utiliser en application topique, des produits déodorants contenant des substances actives de type anti-transpirant, de type bactéricide ou du type absorbeur d'odeurs pour diminuer, voire supprimer, les odeurs axillaires généralement désagréables et/ou pour absorber la sueur.

Les substances anti-transpirantes qui ont pour effet de réduire ou supprimer le flux sudoral sont généralement constituées de sels d'aluminium.

Bien des types différents de compositions anti-transpirantes ont été décrits dans la littérature et sont apparus sur le marché sous des formes telles que des gels, des sticks, des crèmes, des roll-ons ou des aérosols.

Les sels actifs déodorants ou anti-transpirants en particulier les sels hydrosolubles anti-transpirants sont difficilement formulables dans les supports émulsions comprenant une phase aqueuse. L'acidité notamment des actifs anti-transpirants, le taux de sels important font que la plupart des émulsions les contenant ne sont pas stables ou sont difficilement stables. De plus, les actifs déodorants sous forme de sels sont souvent collants (tacky) lors du séchage sur la peau.

Les émulsions déodorantes ou anti-transpirantes classiquement utilisées sont du type huile-dans-eau constituées d'une phase huileuse dispersée dans une phase aqueuse continue et contiennent généralement des émulsionnants non-ioniques. Ce type de support présente l'inconvénient d'avoir des temps de séchage long et d'apporter un toucher collant lors du séchage sur la peau.
Les émulsions du type eau dans huile comprenant une phase aqueuse dispersée dans une phase huileuse continue sont minoritaires dans le domaine des déodorants et des anti-transpirants.

On connaît les émulsions déodorantes ou antitranspirantes eau-dans-huile de type eau-dans-silicone. Ces émulsions contiennent des tensioactifs siliconés et les huiles de silicones sont les constituants majoritaires de la phase huile. Le toucher de ces émulsions reste caractéristique des silicones. On connaît notamment dans la demande de brevet DE 10210461 des compositions antitranspirantes eau-dans-huile riches en phase aqueuse (au moins 70%) et contenant un émulsionnant non-ionique du type dérivé alkylé de polyéthylène glycol (par exemple POLYGLYCERYL-3 ISOSTEARATE) ou du type dérivé alkylé de polyglycérol (par exemple : PEG-30 DIPOLYHYDROXYSTEARATE). Ces émulsions ont l'inconvénient d'être préparées à chaud (75°C).

On connaît dans la demande de brevet CA 1076030 des aérosols antitranspirants sous forme d'émulsion eau-dans-huile contenant un émulsionnant non-ionique est de type polyglycerol-4-oleate.

On connaît dans la demande de brevet GB 2 113 706 des aérosols anti-transpirants sous forme d'émulsion eau-dans-huile contenant un émulsionnant non-ionique en particulier le sorbitan sesquioléate et un mélange d'huile constituée d'un hydrocarbure et d'une huile émolliente.

Ces émulsions eau-dans-huile de l'art antérieur présentent au moins l'un des inconvénient suivants :
- elles n'apportent pas de fraîcheur à l'application.
- elles laissent un film gras sur la peau
- elles ont des problèmes de stabilité en présence d'actifs déodorants sous forme de sel.
- elles ont tendance à diminuer ou inhiber l'activité des sels actifs déodorants.
Il existe donc le besoin de rechercher de nouvelles formulations déodorantes ou anti-transpirantes sous forme d'émulsion eau-dans-huile contenant au moins un sel actif déodorant ou anti-transpirant qui ne présente pas les inconvénients présentés ci-dessus. La demanderesse a découvert de manière surprenante et inattendue que l'utilisation d'un émulsionnant oligomère ou un polymère dérivé de polyoléfine, comportant au moins une partie polaire constituée de polyalkylène glycols, de polyalkylène imines, d'acides ou de diacides carboxyliques, de leurs anhydrides ou dérivés, et leurs mélanges et comportant une partie apolaire polyoléfinique comprenant au moins 40 atomes de carbone dans une émulsion eau-dans-huile contenant au moins un sel actif déodorant ou anti-transpirant permettait d'obtenir des produits déodorants ou anti-transpirants stables dans le temps même avec des taux élevés de sel actif déodorant ou anti-transpirant. Ces produits présentent une bonne efficacité et produisent sur la peau à l'application, après un séchage rapide, une sensation de frais sur la peau à l'application, un effet peu collant ou non collant, ne produisent pas de film gras ni de phénomène de blanchiment.
L'invention a donc pour objet une composition cosmétique comprenant dans un support du type émulsion eau-dans-huile :
(A) au moins un sel actif déodorant ou anti-transpirant;
(B) au moins un émulsionnant oligomère ou un polymère dérivé de polyoléfine, comportant au moins une partie polaire constituée de polyalkylène glycols, de polyalkylène imines, d'acides ou de diacides carboxyliques, de leurs anhydrides ou dérivés, et leurs mélanges et comportant une partie apolaire polyoléfinique comprenant au moins 40 atomes de carbone.
La présente invention a pour objet également un procédé cosmétique de traitement de la transpiration, consistant à appliquer sur la surface de la peau une quantité efficace d'une telle composition.
Le traitement de la transpiration peut consister aussi bien à la réduction ou la suppression de l'odeur désagréable provoquée par la sueur que la réduction ou la suppression de la sensation d'humidité produite par la sueur.
La présente invention a pour objet également un procédé cosmétique de traitement des odeurs corporelles liées à la transpiration (notamment odeurs axillaires, odeurs des pieds), consistant à appliquer sur la surface de la peau une quantité efficace d'une telle composition.
La présente invention a pour objet également l'utilisation d'au moins un émulsionnant oligomère ou un polymère dérivé de polyoléfine, comportant au moins une partie polaire constituée de polyalkylène glycols, de polyalkylène imines, d'acides ou de diacides carboxyliques, de leurs anhydrides ou dérivés, et leurs mélanges et comportant une partie apolaire polyoléfinique comprenant au moins 40 atomes de carbone pour stabiliser une émulsion eau-dans-huile contenant au moins un sel actif déodorant ou anti-transpirant.

La présente invention a pour objet également l'utilisation d'au moins un émulsionnant oligomère ou un polymère dérivé de polyoléfine, comportant au moins une partie polaire constituée de polyalkylène glycols, de polyalkylène imines, d'acides ou de diacides carboxyliques, de leurs anhydrides ou dérivés, et leurs mélanges et comportant une partie apolaire polyoléfinique comprenant au moins 40 atomes de carbone dans une composition cosmétique sous forme d'émulsion eau-dans-huile contenant au moins un sel actif déodorant ou anti-transpirant dans le but d'améliorer les propriétés sensorielles sur la peau après application de ladite composition.'

On entend dans la présente demande par "propriétés sensorielles sur la peau" , la sensation de frais, l'effet non-collant et/ou l'effet non-gras.

On entend dans la présente demande par «émulsion eau-dans-huile», toute composition comportant une phase aqueuse dispersée dans une phase huileuse continue.

Au sens de la présente invention, on entend par "actif déodorant" toute substance capable de réduire ou supprimer le flux sudoral et/ou d'absorber la sueur humaine et/ou de masquer, absorber, améliorer ou réduire l'odeur désagréable résultant de la décomposition de la sueur humaine par des bactéries.

Au sens de la présente invention, on entend par "actif anti-transpirant" toute substance capable de réduire le flux sudoral et/ou d'absorber la sueur humaine.

Les oligomères et polymères utilisables dans l'invention sont connus dans d'autres domaines. Ainsi, ils sont décrits par exemple dans les documents US-A-5,129,972 et US-A-4,919,179, comme stabilisants d'émulsions explosives.
Par ailleurs, ces composés sont connus comme stabilisants de compositions fertilisantes (voir les documents US-A-5,518,517 et US-A-5,858,055) en vue d'obtenir un relargage contrôlé des substances fertilisantes.
Les oligomères et polymères utilisés dans la composition de l'invention sont constitués d'une partie apolaire polyoléfinique et d'au moins une partie polaire. Ils peuvent présenter une structure de type bloc ou peigne.
La partie apolaire polyoléfinique comprend au moins 40 atomes de carbone et de préférence de 60 à 700 atomes de carbone. Cette partie apolaire peut être choisie parmi les polyoléfines telles que les oligomères, les polymères et/ou les copolymères d'éthylène, d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne. Ces polyoléfines sont hydrogénées ou non.
Par ailleurs, les oligomères ou polymères dérivés de polyoléfine utilisés dans la composition de l'invention comportent au moins une partie polaire. Cette partie polaire confère aux dérivés de polyoléfines des propriétés amphiphiles. Ainsi, ces oligomères ou polymères abaissent la tension interfaciale (eau / huile) d'au moins 10 mN/m quand ils sont présents à une concentration de 0,01% en poids par rapport au poids total de la phase huileuse. Par exemple, la polyoléfine à terminaison succinique commercialisée sous la dénomination Lubrizol 2724 par la société Lubrizol, à une concentration de 0,01% en poids par rapport au poids total de la phase huileuse, abaisse la tension interfaciale de 15 mN/m à l'interface d'une phase aqueuse constituée d'une solution aqueuse à 1% de MgSO₄, et d'une phase huileuse comportant un mélange d'huiles (isohexadécane /polyisobutène hydrogéné/silicone volatile dans un rapport 8/6/4).

La partie polaire des oligomères ou polymères de l'invention peut être anionique, cationique, non ionique, zwitterionique ou amphotère. Elle est constituée de polyalkylène glycols ou de polyalkylène imines, ou encore d'acides ou de diacides carboxyliques, de leurs anhydrides ou de leurs dérivés, et leurs mélanges. Les oligomères ou polymères à partie polaire acide carboxylique peuvent être par exemple issus de la réaction entre une polyoléfine et au moins un acide ou anhydride carboxylique choisi dans le groupe comprenant l'acide maléique, l'anhydride maléique, l'acide fumarique, l'acide itaconique, l'acide citraconique, l'acide mésaconique, l'acide aconitique. De préférence, la partie polaire est constituée par l'acide ou l'anhydride succinique, leurs dérivés esters ou amides, les sels d'ions alcalins, alcalino-terreux ou organiques correspondants, ou bien encore par du polyoxyéthylène.

Les oligomères ou polymères dérivés de polyoxyéthylène peuvent être par exemple choisis parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p.1582-1586).

Les oligomères ou polymères dérivés d'acide ou d'anhydride succinique peuvent être choisis notamment parmi les dérivés polyoléfines d'acide ou d'anhydride succinique décrits dans les brevets US-A-4,234,435, US-A-4,708,753, US-A-5,129,972, US-A-4,931,110, GB-A-2,156,799 et US-A-4,919,179 incorporés ici pour référence. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatique, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753 qui est incorporé ici pour référence.

Comme polyoléfines à terminaison succinique, on peut citer notamment les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol ou les produits Chemcinate 100AF et 2000 de la société CHEMRON

Un autre exemple de tensioactif polymérique utilisable dans l'invention est le produit de la réaction de l'anhydride maléique avec le polyisobutylène, tel que le produit commercialisé sous la dénomination Glissopal SA par la société BASF.

La quantité d'oligomère(s) ou de polymère(s) dans la composition de l'invention peut aller par exemple de 0,1 à 20 % en poids de matière active, de préférence de 0,2 à 10 % en poids et mieux de 0,5 à 5 % en poids par rapport au poids total de la composition. On peut utiliser un ou plusieurs oligomères ou polymères dérivés de polyoléfines.

Parmi les sels actifs déodorants utilisables selon l'invention, on peut citer les actifs anti-transpirants ou astringents. Ils sont choisis de préférence parmi les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes (lorsque l'acide aminé est la glycine )".

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Les sels actifs déodorants conformes à l'invention peuvent être aussi des agents bactériostatiques ou des agents bactéricides comme les sels d'ammonium quaternaires par exemple les sels de cétyltrimethylammonium, les sels de cétylpyridinium

Parmi les autres actifs déodorants sous forme de sel, on peut citer également
- les sels de zinc comme le salicylate de zinc , le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoleate de zinc, glycinate de zinc, carbonate de zinc, citrate de zinc, chlorure de zinc, laurate de zinc, oléate de zinc, orthophosphate de zinc, stéarate de zinc, silicate de zinc, tartrate de zinc, lactate de zinc ou acétate de zinc ;
- les sels de chlorhexidine ;
- les sels de polyhexaméthylène biguanide
- le bicarbonate de sodium.

On utilisera plus particulièrement les sels actifs déodorants hydrosolubles et encore plus particulièrement les sels anti-transpirants et de préférence les sels d'aluminium.

Les actifs déodorants sous forme de sel peuvent être présents dans la composition selon l'invention à raison de 0,1 à 30% en poids par rapport à la composition totale, et de préférence de 10 à 25% en poids.

Les compositions selon l'invention peuvent également contenir d'autres actifs déodorants additionnels comme le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) la chlorhexidine, le monocaprate de diglycérol, le monolaurate de diglycérol , monolaurate de glycérol.

La composition cosmétique selon l'invention peut se présenter sous forme plus ou moins épaissie distribuée en tube ou en grille ; sous forme de roll-on conditionnée sous forme de bille ; sous forme de stick ; sous forme de spray ou d'aérosol et contenir à cet égard les ingrédients généralement utilisés dans ce type de produits et bien connus de l'homme de l'art.

La phase grasse conforme aux émulsions eau-dans-huile selon l'invention est présente dans une quantité allant de préférence de 3 à 80% en poids par rapport au poids total de l'émulsion.

La phase grasse comprend en général un ou plusieurs composés hydrophobes qui rendent ladite phase non-miscible dans l'eau.

La phase grasse contient de préférence une ou plusieurs huiles émollientes hydrocarbonées non-siliconées, volatiles ou non-volatiles.

Parmi les huiles émollientes hydrocarbonées utilisables dans la présente invention, on peut citer par exemple : les dérivés hydrocarbonés liquides comme le squalane, les huiles minérales et les polyisobutène hydrogénés et plus particulièrement les polydécenes, les paraffines, les huiles hydrocarbonées à chaine ramifiée qui comportent de préférence de 6 à 20 et mieux de 6 à 18 atomes de carbone et peuvent être choisies par exemple dans le groupe comprenant l'isohexadécane, l'isododécane, les isoparaffines, les alcools gras liquides à température ambiante tels que l'alcool isostéarique ou l'octyl dodécanol ; les esters aliphatiques d'alcools en C3-C18 avec des acides en C₃-C₁₈ comme isopropyl myristate, lauryl myristate, isopropyl palmitate , diisopropyl sebacate, diisopropyl adipate ; les esters aromatiques de l'acide benzoïque avec des alcools en C₁₂-C₁₈ et leurs mélanges comme les alkylbenzoates en C8-C18 ; les éthers d'alcools gras aliphatiques tels que les éthers d'alcool myristique comme le PPG-3 myristyl éther et les alkyl(C₁-C₄)éthers de polyglycols comme le PPG-4 butyl éther. Les huiles végétales telles que huiles de noyaux d'abricot, l'huiles d'avocat, l'huile de noix de macadamia, l'huile de tournesol, l'huile d'olive et l'huile de soja.

Les huiles émollientes selon l'invention peuvent être choisies parmi des huiles siliconées du type silicone volatile ou non-volatiles.

Les silicones volatiles sont définies de façon connue comme des composés volatils à température ambiante. (20-25°C). Elles présentent en général une vapeur de pression à 25°C allant de 1 ou 10 Pa à 2kPa.

On peut citer parmi ces composés les silicones volatiles cycliques et linéaires du type diméthylsiloxane dont les chaines comprennent de 3 à 9 résidus siliconés.

De préférence on choisit les cyclométhicones tétramère (D₄), pentamère (D₅) ou hexamère (D₆).

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de «®Dow Corning 245 Fluid» ; les polyalkylarylsiloxanes comme par exemple les polyméthylphénylsiloxanes, commercialisés par la société Dow Corning sous le nom de «® Dow Corning 556 Fluid» ; les copolymères polyéther et siloxane, comme par exemple les diméthicones copolyols.

Afin d'améliorer l'homogénéité du produit, on peut utiliser des agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom ®TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ®ORGASOL par la société Atochem ; les microsphères à base de copolymères acryliques, telles que celles en copolymère diméthacrylate d'éthylène glycol/ methacrylate de lauryle vendues par la société Dow Corning sous la dénomination de ®POLYTRAP ; les microsphères de polyméthacrylate de méthyle, commercialisées sous la dénomination ®MICROSPHERE M-100 par la société Matsumoto ou sous la dénomination ®COVABEAD LH85 par la société Wackherr ; les poudres de copolymère éthylène-acrylate, comme celles commercialisées sous la dénomination ®FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination ®EXPANCEL par la société Kemanord Plast sous les références ®551 DE 12 (granulométrie d'environ 12 µm et masse volumique 40 kg/m3), ®551 DE 20 (granulométrie d'environ 30 µm et masse volumique 65 kg/m3), ®551 DE 50 (granulométrie d'environ 40 µm), ou les microsphères commercialisées sous la dénomination ®MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination ®TOSPEARL par la société Toshiba Silicone, notamment TOSPEARL 240 ; les poudres d'aminoacides telles que la poudre de Lauroyllysine commercialisée sous la dénomination ®AMIHOPE LL-11 par la Société Ajinomoto.

La phase aqueuse desdites émulsions contient de l'eau et en général d'autres solvants solubles ou miscibles dans l'eau. Les solvants solubles ou miscibles dans l'eau comprennent les mono alcools à chaîne courte par exemple en C1-C4 comme l'éthanol, l'isopropanol ; les diols ou les polyols comme l'éthylèneglycol, le 1,2-propylèneglycol, ie 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

La quantité d'eau varie de préférence de 20 à 85 % en poids par rapport au poids total de la composition.

La composition cosmétique selon l'invention peut comprendre en outre des adjuvants cosmétiques choisis parmi les adoucissants, les antioxydants, les opacifiants, les stabilisants, les agents hydratants, les vitamines, les parfums, des bactéricides, les conservateurs, les polymères, les parfums, les agents épaississants, ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la ® Bentone Gel MIO vendue par la société NL INDUSTRIES ou la ®Veegum Ultra, vendue par la société POLYPLASTIC.

Les quantités de ces différents constituants pouvant être présents dans la composition cosmétique selon l'invention sont celles classiquement utilisées dans les crèmes, sticks-grilles et roll-ons anti-transpirants.

Les compositions selon l'invention peuvent également contenir également un ou plusieurs autres agents structurants ou gélifiants de la phase huileuse tels que ceux évoqués précédemment dans la description tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'―dibutytsuccinamide) ;les organopolysiloxane élastomères tels que ceux décrits dans la demande WO97/44010.

La composition selon l'invention peut encore être pressurisée et être conditionnée dans un aérosol; elle contient alors les propulseurs généralement utilisés dans ce type de produits et bien connus de l'homme de l'art, comme par exemple le diméthyléther ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale ®DYMEL 152 A par la société DUPONT.

On peut également utiliser en tant qu'agent propulseur le gaz carbonique, le protoxyde d'azote, l'azote ou l'air comprimé.

L'agent propulseur représente alors de préférence de 20 à 85% en poids du poids

Des exemples concrets, mais nullement limitatifs, illustrant l'invention vont maintenant être donné.

### Tests comparatifs de stabilité

On observe la stabilité dans le temps de différentes émulsions eau-dans-huile contenant un sel actif anti-transpirant (chlorhydrate d'aluminium) en effectuant plusieurs cycles de conservation comprenant des montées et descentes périodiques de température

Chaque cycle est divisé en 4 périodes :
6 heures à 20°C
6 heures de descentes jusqu'à -20°C
6 heures à -20°C
6 heures de montée à +20°C.

Chaque formule est évaluée macroscopiquement après chaque cycle.

| **Ingrédients** | Exemple A (art antérieur exemple 1 de la demande DE 10210461) |
|---|---|
| PEG-30 dipolyhydroxysteatate | 1,0 g |
| Dicapryléther | 9,0 g |
| Glycérine | 5,0 g |
| Sulfate de magnésium | 0,6 g |
| Chlorhydrate d'Aluminium (solution aqueuse à 50%) | 10,0 g |
| Parfum | 1,0 g |
| Eau | 73,4 g |
| Stabilité en cycles | Instable après 1 cycle |

L'exemple A a été préparé selon le procédé décrit dans le document DE10210461

| **Ingrédients** | **Exemple B** **(hors invention)** | **Exemple C** **(hors invention)** | **Exemple D** **(hors invention)** | **Exemple 1** **(Roll-on)** | **Exemple 2** **(Roll-on)** |
|---|---|---|---|---|---|
| PEG-30 dipolyhydroxysteatate | 1,90 g | 1,90 g | - | - | - |
| Polyglyceryl-3-isostearate (and) Sorbitan Isosterate | - | - | 1,90 g | | |
| Polyisobutylene à terminaison succinique esterifiée, sel de diéthyléthanolamine (Chemccinnate 2000) | - | - | - | 1,90 g | 1,90 g |
| Squalane | 2,16 g | 2,16 g | 2,16 g | 2,16 g | 2,16 g |
| Palmitate d'ethylhexyle | 4,65 g | - | - | 4,65 g | - |
| Isohexadécane | - | 4,65 g | 4,65 g | | 4,65g |
| Cyclohexasiloxane | 2,33 g | 2,33 g | 2,33 g | 2,33 g | 2,33 g |
| PRUNUS ARMENIACA (APRICOT) KERNEL OIL | 1,32 g | 1,32 g | 1,32 g | 1,32 g | 1,32 g |
| Glycérine | 5,00 g | 5,00 g | 5,00 g | 5,00 g | 5,00 g |
| Chlorhydrate d'Aluminium (solution aqueuse à 50%) | 40,00 g | 40,00 g | 40,00 g | 40,00 g | 40,00 g |
| Conservateurs | 0,99 g | 0,99 g | 0,99 g | 0,99 g | 0,99 g |
| Eau | 41,65 g | 41,65 g | 41,65 g | 41,65 g | 41,65 g |
| **Stabilité en cycles** | **Instable après 1 cycle** | **Instable après 1 cycle** | **Instable après 2 cycles** | **Stable après 10 cycles** | **Stable après 10 cycles** |

### Procédés de formulation :

### Exemples B, C et D

On homogénéise la phase grasse à 75°C. On homogénéise séparément sous agitation les constituants de la phase aqueuse à température ambiante. On disperse la phase aqueuse dans la phase huileuse, sous agitation Rayneri à 600rpm pendant 10 minutes et à température ambiante.

### Exemples 1 et 2 (Roll-on)

On procède dans les mêmes conditions que les exemples B, C et D mais les constituants de la phase grasse sont mélangés à 45°C pour obtenir une phase homogène.

Les émulsions eau-dans-huile 1 et 2 selon l'invention contenant le dérivé de polyoléfine comportant au moins une partie polaire sont stables en cycle comparativement aux exemples A, B, C et D de l'art antérieur contenant des tensio-actifs non-ioniques polyglycérolés ou polyoxyéthylénés.

### Evaluation cosmétique :

Les émulsions eau-dans-huile 1 et 2 selon l'invention sont fraîches à l'application et n'apportent pas de film gras pendant et après l'application des produits comparativement à celles de l'art antérieur.

### Exemples 3 et 4 : Crèmes anti-transpirantes

| **Ingrédients** | **Exemple 3** | **Exemple 4** |
|---|---|---|
| Polyisobutylene à terminaison succinique esterifiée, sel de diéthyléthanolamine (Lubrizol 5603) | 1,92 g | 1,92 g |
| Squalane | 2,16 g | 2,16 g |
| Palmitate d'ethylhexyle | - | 4,65 |
| Isohexadecane | 4,65 g | - |
| Cyclohexasiloxane | 2,33 g | 2,33 g |
| Glycérine | 5,00 g | 5,00 g |
| Chlorhydrate d'Aluminium (solution aqueuse à 50%) | 40,00g | 40,00g |
| PRUNUS ARMENIACA (APRICOT) KERNEL OIL | 1,32 g | 1,32 g |
| Conservateurs | 0,99 g | 0,99 g |
| Eau | 41,63 g | 41,63 g |
| Viscosité 24°C (mobil 3, 200s-1) | 5,1 Pa.s | 4,5 Pa.s |

### Procédé de formulation :

On homogénéise la phase grasse la phase grasse à 45°C. Les constituants de la phase aqueuse sont mélangés séparément sous agitation à température ambiante jusqu'à homogénéisation On disperse la phase aqueuse dans la phase huileuse, sous agitation Rayneri à 600rpm pendant 10 minutes.

Les formules se présentent sous forme d'une crème souple, lisse et brillante. Les formules sont stables 2 mois de 4 à 45°C.

### Exemple 5 : Aérosol anti-transpirant :

| **Ingrédients** | **Exemple 5** |
|---|---|
| Polyisobutylene à terminaison succinique esterifiée, sel de diéthyléthanolamine (Lubrizol 5603) | 0,96 g |
| Squalane | 1,08 g |
| Isohexadecane | 2,33 g |
| Cyclohexasiloxane | 1,17 g |
| PRUNUS ARMENIACA (APRICOT) KERNEL OIL | 0,66 g |
| Cyclopentasiloxane (and) Disteadimonium Hectorite (and) Denatured alcohol | 1,50 g |
| Glycérine | 2,50 g |
| Chlorhydrate d'Aluminium (solution aqueuse à 50%) | 20,00 g |
| Eau | 19,81 g |
| Isobutane | 50,00 g |

### Procédé de formulation :

Préparation du jus Le procédé de fabrication du jus est le même que celui des exemples de l'invention ci-dessus.

Pressurisation Le jus est pressurisé à l'isobutane selon les techniques classiques.

On observe ensuite le phénomène de résidu blanc après application avec l'aérosol selon l'invention et on le compare avec à la formule Clearex® d'Unilever connue pour être peu blanchissante à l'application.

On pulvérise, à 15cm de distance, environ de 0,3 gr de jus (quantité hors propulseur) sur un support BIOSKIN ( PLATE BLACK) vendu par la société MAPRECOS.SA.

Le substrat est ensuite placé dans une boîte à lumière. On filme la zone traitée pendant 4 heures au moyen d'une caméra noir et blanc MICROVISION à raison de 1 photographie toutes les 10 secondes. On mesure et on calcule au moyen d'un logiciel la nuance de gris observée sur le support en fonction du temps.
Cette nuance de gris représente le résidu blanc sur le substrat après application. Plus la valeur est élevée plus la nuance est blanche.

Les valeurs de gris mesurées pour chaque aérosol, sont représentées sur le tableau suivant :

| **Formules** | **Niveau de gris après 1 heure** |
|---|---|
| Exemple 5 (invention) | 65 |
| Aérosol Clearex® | 73 |

La formule est moins blanchissante que la référence du marché.

### Exemple 6 : Stick antitranspirant:

| **Ingrédients** | **Exemple6** | **Exemple 7** |
|---|---|---|
| Polyisobutylene à terminaison succinique esterifiée, sel de diéthyléthanolamine (Chemcinnate 2000) | 1,90 g | 1,90g |
| Isohexadecane | 10,60 g | 10,60 g |
| Huile de ricin hydrogénée | | 3,00 g |
| Alcool stéarylique | 13,00 g | 11,00 g |
| Chlorhydrate d'Aluminium (solution aqueuse à 50%) | 40,00g | 40,00g |
| Glycérine | 5,00 g | 5,00 g |
| Eau | 29,5 g | 28,5 g |

### Procédé de formulation :

On fait fondre, à environ 80°C, la phase grasse jusqu'à homogénéisation du milieu. Les constituants de la phase aqueuse sont mélangés séparément sous agitation à température ambiante jusqu'à homogénéisation. Sous agitation Rayneri à 400rpm, on ajoute goutte à goutte la phase aqueuse sur la phase huileuse.(20g d'eau/minute) On laisse sous agitation Rayneri 20minutes à 1000rpm. On coule l'émulsion, à chaud, dans des sticks et refroidir rapidement à +4°c. Le coulage est réalisé environ à 5°c au-dessus du point de ramollissement de chaque stick.

## Revendications

1. Composition cosmétique comprenant dans un support du type émulsion eau-dans-huile :
(A) au moins un sel actif déodorant ;
(B) au moins un émulsionnant oligomère ou un polymère dérivé de polyoléfine, comportant au moins une partie polaire constituée de polyalkylène glycols, de polyalkylène imines, d'acides ou de diacides carboxyliques, de leurs anhydrides ou dérivés, et leurs mélanges et comportant une partie apolaire polyoléfinique comprenant au moins 40 atomes de carbone.

2. Composition selon la revendication 1, **caractérisée en ce que** l'émulsionnant oligomère ou le polymère dérivé de polyoléfine comporte une partie apolaire polyoléfinique comprenant de 60 à 700 atomes de carbone.

3. Composition selon la revendication 2, **caractérisée en ce que** la partie apolaire polyoléfinique est choisie parmi les oligomères, les polymères et/ou les copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne.

4. Composition selon l'une quelconque des revendications précédentes, **caractérisée en ce que** l'oligomère ou le polymère dérivé de polyoléfine abaisse la tension interfaciale d'au moins 10 mN/m quand ledit oligomère ou polymère est présent à une concentration de 0,01% en poids par rapport au poids de phase huileuse.

5. Composition selon l'une quelconque des revendications précédentes, **caractérisée en ce que** la partie polaire peut être anionique, cationique, non ionique, zwitterionique ou amphotère.

6. Composition selon l'une quelconque des revendications précédentes, **caractérisée en ce que** la partie polaire est choisie dans le groupe comprenant le polyoxyéthylène, l'acide ou l'anhydride succinique et leurs dérivés.

7. Composition selon l'une quelconque des revendications précédentes, **caractérisée en ce que** l'oligomère ou le polymère dérivé de polyoléfine est issu de la réaction entre un dérivé de polyoléfine et au moins un acide choisi dans le groupe comprenant l'acide maléique ; l'anhydride maléique; l'acide fumarique ; l'acide itaconique; l'acide citraconique ; l'acide mésaconique , l'acide aconitique ; leurs dérivés et leurs mélanges.

8. Composition selon l'une quelconque des revendications précédentes, **caractérisée en ce que** l'oligomère ou polymère dérivé de polyoléfine est un polyisobutylène à terminaison succinique éventuellement modifiée.

9. Composition selon l'une quelconque des revendications précédentes, **caractérisée en ce que** l'oligomère ou polymère dérivé de polyoléfine est le produit de la réaction de l'anhydride maléique avec le polyisobutylène.

10. Composition selon l'une quelconque des revendications précédentes, **caractérisée en ce que** la quantité d'oligomère(s) ou polymère(s) dérivé(s) de polyoléfine va de 0,1 à 20 % en poids de matière active par rapport au poids total de la composition.

11. Composition selon l'une quelconque des revendications précédentes, où le sel actif déodorant est choisi parmi les sels anti-transpirants.

12. Composition selon la revendication 11, où le sel anti-transpirant est choisi parmi les sels d'aluminium et/ou de zirconium , les complexes d'hydroxychlorure de zirconium et d'hydroxychlorure d'aluminium avec un acide aminé

13. Composition selon la revendication 12, où l'actif anti-transpirant est choisi parmi le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

14. Composition selon la revendication 12, où l'actif anti-transpirant est choisi parmi l'aluminium zirconium octachlorohydrate, l'aluminium zirconium pentachlorohydrate, l'aluminium zirconium tetrachlorohydrate, l'aluminium zirconium trichlorohydrate.

15. Composition selon la revendication 12, où l'actif anti-transpirant est choisi parmi les complexes d'hydroxychlorure de zirconium et d'hydroxychlorure d'aluminium avec la glycine (ZAG).

16. Composition selon la revendication 15, où l'actif anti-transpirant est choisi parmi les complexes aluminium zirconium octachlorohydrex glycine, aluminium zirconium pentachlorohydrex glycine, aluminium zirconium tetrachlorohydrex glycine et aluminium zirconium trichlorohydrex glycine.

17. Composition selon l'une quelconque des revendications 1 à 10, où le sel actif déodorant est choisi parmi les agents bactériostatiques ou des agents bactéricides.

18. Composition selon la revendication 17, où le sel actif déodorant est choisi parmi les sels d'ammonium quaternaire.

19. Composition selon la revendication 18, où le sel actif est choisi parmi les sels de cétyltrimethylammonium, les sels de cétylpyridinium.

20. Composition selon l'une quelconque des revendications 1 à 10, où le sel actif est choisi
- les sels de zinc ;
- les sels de chlorhexidine ;
- les sels de polyhexaméthylène biguanide
- le bicarbonate de sodium.

21. Composition selon la revendication 20, où le sel de zinc est choisi parmi le salicylate de zinc , le phénolsulfonate de zinc, le pyrrolidone carboxylate de zinc, le sulfate de zinc, le chlorure de zinc, le lactate de zinc, le gluconate de zinc, ricinoleate de zinc, glycinate de zinc, carbonate de zinc, citrate de zinc, chlorure de zinc, laurate de zinc, oléate de zinc, orthophosphate de zinc, stéarate de zinc, silicate de zinc, tartrate de zinc, lactate de zinc ou acétate de zinc.

22. Composition selon l'une quelconque des revendications 1 à 21, où le sel actif déodorant est hydrosoluble.

23. Composition selon la revendications 22, où le sel actif déodorant hydrosoluble est le chlorhydrate d'aluminium sous forme activée ou non activée.

24. Composition selon l'une quelconque des revendications 1 à 23, où le sel actif déodorant est présent de 0,1 à 30% en poids par rapport à la composition totale, et de préférence de 10 à 25% en poids.

25. Composition selon l'une quelconque des revendications 1 à 24 contenant en plus au moins un actif déodorant additionnel qui n'est pas sous forme de sel.

26. Composition selon la revendication 25, où l'actif déodorant additionnel est choisi parmi le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) la chlorhexidine, le monocaprate de diglycérol, le monolaurate de diglycérol , monolaurate de glycérol.

27. Composition selon l'une quelconque des revendications 1 à 26, **caractérisée par le fait qu'**elle se présente sous forme plus ou moins épaissie distribuée en tube ou en grille ; sous forme de roll-on conditionnée sous forme de bille ; sous forme de stick ; sous forme de spray ou d'aérosol.

28. Composition selon l'une quelconque des revendications 1 à 27, **caractérisée par le fait que** la phase huileuse contient une ou plusieurs huiles émollientes non-siliconées hydrocarbonées, volatiles ou non-volatiles.

29. Composition selon l'une quelconque des revendications 1 à 28, **caractérisée par le fait qu'**elle contient en plus au moins un agent de suspension.

30. Composition selon l'une quelconque des revendications 1 à 30, **caractérisée par le fait qu'**elle contient en plus au moins une poudre organique.

31. Composition selon l'une quelconque des revendications 1 à 30, **caractérisée par le fait qu'**elle contient en plus des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants.

32. Composition selon l'une quelconque des revendications 1 à 31, **caractérisée par le fait qu'**elle contient en plus un ou plusieurs agents structurants ou gélifiants de la phase huileuse

33. Composition selon l'une quelconque des revendications 1 à 32, **caractérisée par le fait qu'**elle est pressurisée et conditionnée dans un aérosol et qu'elle contient un ou plusieurs agents propulseurs.

34. Procédé cosmétique de traitement de la transpiration, consistant à appliquer sur la surface de la peau une quantité efficace d'une composition telle que définie selon l'une quelconque des revendications 1 à 33.

35. Procédé cosmétique de traitement des odeurs corporelles liées à la transpiration, consistant à appliquer sur la surface de la peau une quantité efficace d'une telle composition telle que définie selon l'une quelconque des revendications 1 à 33.

36. Utilisation d'au moins un émulsionnant oligomère ou un polymère dérivé de polyoléfine, comportant au moins une partie polaire selon l'une quelconque des revendications 1 à 9 pour stabiliser une émulsion eau-dans-huile contenant au moins un sel actif déodorant.

37. Utilisation d'au moins un émulsionnant oligomère ou un polymère dérivé de polyoléfine, comportant au moins une partie polaire selon l'une quelconque des revendications 1 à 9 dans une composition cosmétique sous forme d'émulsion eau-dans-huile contenant au moins un sel actif déodorant ou anti-transpirant dans le but d'améliorer les propriétés sensorielles sur la peau après application de ladite composition.

## Claims

1. Cosmetic composition comprising, in a vehicle of the water-in-oil émulsion type:
(A) at least one deodorant active salt;
(B) at least one polyolefin-derived emulsifying oligomer or polymer comprising at least one polar part, composed of polyalkylene glycols, of polyalkylene-imines, of carboxylic or dicarboxylic acids, of their anhydrides or derivatives, and their mixtures, and comprising a nonpolar polyolefinic part comprising at least 40 carbon atoms.

2. Composition according to Claim 1, **characterized in that** the polyolefin-derived emulsifying oligomer or polymer comprises a nonpolar polyolefinic part comprising from 60 to 700 carbon atoms.

3. composition according to Claim 2, **characterized in that** the nonpolar polyolefinic part is chosen from oligomers, polymers and/or copolymers of ethylene, propylane, 1-butene, isobutene, 1-pentene, 2-methyl-1-butene, 3-methyl-1-butene, 1-haxene, 1-heptene, 1-octene, 1-decena, 1-undecene, 1-dodecene, 1-tridecene, 1-tetradecene, 1-pentadecene, 1-hexadecene, 1-haptadecene and 1-octadecene.

4. Composition according to any one of the preceding claims, **characterized in that** the polyolefin-derived oligomer or polymer lowers the interfacial tension by at least 10 mN/m when the said oligomer or polymer is present at a concentration of 0.01% by weight with respect to the weight of oily phase.

5. composition according to any one of the preceding claims, **characterized in that** the polar part can be anionic, cationic, nonionic, zwitterionic or amphoteric.

6. Composition according to any one of the preceding claims, **characterized in that** the polar part is chosen from the group consisting of polyoxyethylene, succinic acid or anhydride, and their derivatives.

7. Composition according to any one of the preceding claims, **characterized in that** the polyolefin-derived oligomer or polymer results from the reaction between a polyolefin derivative and at least one acid chosen from the group consisting of maleic acid, maleic anhydride, fumaric acid, itaconic acid, citraconic acid, mesaconic acid, aconitic acid, their derivatives and their mixtures.

8. composition according to any one of the preceding claims, **characterized in that** the polyolefin-derived oligomer or polymer is a polyisobutylene comprising an optionally modified succinic ending.

9. Composition according to any one of the preceding claims, **characterized in that** the polyolefin-derived oligomer or polymer is the product of the reaction of maleic anhydride with polyisobutylene.

10. Composition according to any one of the preceding claims, **characterized in that** the amount of polyolefin-derived oligomer(s) or polymer(s) ranges from 0.1 to 20% by weight of active material with respect to the total weight of the composition.

11. composition according to any one of the preceding claims, where the deodorant active salt is chosen from antiperspirant salts.

12. composition according to claim 11 where the antiperspirant salt is chosen from aluminium and/or zirconium salts; or complexes of zirconium chlorohydrate and of aluminium chlorohydrate with an amino acid.

13. composition According to claim 12 where the antiperspirant active principle is chosen from aluminium chlorohydrate in the activated or nonactivated form, aluminium chlorohydrex, the aluminium chlorohydrex polyethylene glycol complex, the aluminium chlorohydrex propylene glycol complex, aluminium dichlorohydrate, the aluminium dichlorohydrex polyethylene glycol complex, the aluminium dichlorohydrex propylene glycol complex, aluminium sesquichlorohydrate, the aluminium sesquichlorohydrex polyethylene glycol complex, the aluminium sesquichlorohydrex propylene glycol complex, or aluminium sulphate buffered by sodium aluminium lactate.

14. Composition according to Claim 12 where the antiperspirant active principle is chosen from aluminium zirconium octachlorohydrate, aluminium zirconium pentachlorohydrate, aluminium zirconium tetrachlorohydrate or aluminium zirconium trichlorohydrate.

15. Composition according to Claim 12, where the antiperspirant active principle is chosen from complexes of zirconium chlozohydrate and of aluminium chlorohydrate with glycine (ZAG).

16. Composition according to Claim 15, where the antiperspirant active principle is chosen from aluminium zirconium octachlorohydrex glycine, aluminium zirconium pentachlorohydrex glycine, aluminium zirconium tetrachlorohydrex glycine and aluminium zirconium trichlorohydrex glycine complexes.

17. Composition according to any one of Claims 1 to 10, where the deodorant active salt in chosen from bacteriostatic agents or bactericidal agents.

18. composition according to claim 17 where the deodorant active salt is chosen from quaternary ammonium salts.

19. Composition according to claim 18, where the active salt is chosen from cetylerimethylammonium salts or Cetylpyridinium salts.

20. Composition according to any one of Claims 1 to 10, where the active salt is chosen from :
- zinc salts ;
- chlorohexidine salts;
- polyhexamethylene biguanide salts;
- sodium bicarbonate.

21. composition according to claim 20, where the zinc salt is chosen from zinc salicylate, zinc phenolsulphonate, zinc pyrrolidonecarboxylate, zinc sulphate, zinc chloride, zinc lactate, zinc gluconate, zinc ricinoleate, zinc glycinate, zinc carbonate, zinc citrate, zinc laurate, zinc oleate, zinc orthophosphate, zinc atearate, zinc silicate, zinc tartrate or zinc acetate.

22. Composition according to any one of claims 1 to 21, where the deodorant active salt is water-soluble.

23. Composition according to Claim 22, where the water-soluble deodorant active salt is aluminium chlorohydrate in the activated or nonactivated form.

24. composition according to any one of Claims 1 to 23 where the deodorant active salt is present in a proportion of 0.1 to 30% by weight with respect to the total composition and preferably of 10 to 25% by weight.

25. Composition according to any one of claims 1 to 24 furthermore comprising at least one additional deodorant active principle which is not in the salt form.

26. Composition according to claim 25 where the additional deodorant active principle is chosen from 2,4,4'-trichloro-2'-hydxoxydiphenyl ether (Triclosan), 2,4-dichloro-2'-hydroxydiphenyl ether, 3',4',5'-trichlorosalicylanilide, 1-(3',4'-dichlorophenyl)-3-(4'-chlorophenyl) urea (Triolocarban) or 3,7,11-trimethyldodeca-2,5,10-trienol (Farnesol), chlorhexidine, diglycerol monocaprate, diglycerol monolaurate or glycerol monolaurate.

27. Composition according to any one of Claims 1 to 26, **characterized in that** it is provided in the more or less thickened form distributed in a tube or in a twist Stick; in the form of a roll-on packaged in the ball form; in the stick form; or in the spray or aerosol form.

28. Composition according to any one of Claims 1 to 27, **characterized in that** the oily phase comprises one or more volatile or non-volatile non-silicone hydrocarbon emollient oils.

29. Composition according to any one of claims 1 to 28, **characterized in that** it additionally comprises at least one suspending agent.

30. Composition according to any one of Claims 1 to 30, **characterized in that** it additionally comprises at least one organic powder.

31. Composition according to any one of claims 1 to 30, **characterized in that** it additionally comprises cosmetic adjuvants chosen from waxes, softeners, antioxidants, opacifiers, stabilizers, moisturizing agents, vitamins, fragrances, bactericides, preservatives, polymers or thickening agents.

32. Composition according to any one of claims 1 to 31 **characterized in that** it additionally comprises one or more structuring or gelling agents for the oily phase.

33. Composition according to any one of Claims 1 to 32, **characterized in that** it is pressurized and packaged in an aerosol and **in that** it comprises one or more propellants.

34. Cosmetic process for the treatment of perspiration, which consists in applying, to the surface of the skin, an effective amount of a composition as defined according to any one of Claims 1 to 33.

35. Cosmetic process for the treatment of body odours related to perspiration, which consists in applying, to the surface of the skin, an effective amount of such a composition as defined according to any one of Claims 1 to 33.

36. Use of at least one polyolefin-derived emulsifying oligomer or polymer comprising at least one polar part according to any one of Claims 1 to 9 for stabilizing a water-in-oil emulsion comprising at least one deodorant active salt.

37. Use of at least one polyolefin-derived emulsifying oligomer or polymer comprising at last one polar part according to any one of claims 1 to 9 in a cosmetic composition in the form of a water-in-oil emulsion comprising at least one deodorant or antiperspirant active salt for the purpose of improving the sensory properties on the skin after application of the said composition.

## Patentansprüche

1. Kosmetische Zusammensetzung, die in einem Träger vom Typ einer Wasser-in-Öl-Emulsion enthält:
(A) mindestens ein wirksames desodorierendes Salz;
(B) mindestens einen oligomeren Emulgator oder ein von einem Polyolefin abgeleitetes Polymer mit mindestens einem polaren Teil, der aus Polyalkylenglycolen, Polyalkyleniminen, Carbonsäuren oder Dicarbonsäuren, ihren Anhydriden oder Derivaten, sowie ihren Gemischen besteht, und mit einem apolaren poyolefinischen Teil aufweist, der mindestens 40 Kohlenstoffatome enthält.

2. Zusammensetzung nach Anspruch 1, **dadurch gekennzeichnet, dass** der oligomere Emulgator oder das von einem Polyolefin abgeleitete Polymer einen apolaren polyolefinischen Teil aufweist, der 60 bis 700 Kohlenstoffatome enthält.

3. Züsammensetzung nach Anspruch 2, **dadurch gekennzeichnet, dass** der apolare polyolefinische Teil unter den oligomeren, den Polymeren und/oder den Copolymeren von Ethylen, Propylen, 1-Buten, Isobuten, 1-Penten, 2-Methyl-1-buten, 3-Methyl-1-buten, 1-Hexen, 1-Hepten, 1-Octen, 1-Decen, 1-Undecen, 1-Dodecen, 1-Tridecen, 1-Tetradecen, 1-Pentadecen, 1-Hexadecen, 1-Heptadecen und 1-Octadecen ausgewählt ist.

4. Zusammensetzung nach einem der vorhergehenden Ansprüche, **dadurch gekennzeichnet, dass** das Oligomer öder das von einem Polyolefin abgeleitete Polymer die Grenzflächenspannung um mindestens 10 mN/m absenkt, wenn das Oligomer oder das Polymer in einer Konzentration von 0,0 1 Gew.-%, bezogen auf das Gewicht der Ölphase, vorliegt.

5. Zusammensetzung nach einem der vorhergehenden Ansprüche, **dadurch gekennzeichnet, dass** der polare Teil anionisch, kationisch, nichtionisch, zwitterionisch oder amphoter sein kann.

6. Zusammensetzung nach einem der vorhergehenden Ansprüche, **dadurch gekennzeichnet, dass** der polare Teil unter Polyoxyethylen, Bernsteinsäure oder Bernsteinsäureanhydrid und ihren Derivaten ausgewählt ist.

7. Zusammensetzung nach einem der vorhergehenden Ansprüche, **dadurch gekennzeichnet, dass** das Oligomer oder das von einem Polyolefin abgeleitete Polymer bei der Reaktion eines Polyolefinderivats und mindestens einer Säure gebildet wird, die unter Maleinsäure, Maleinsäureanhydrid; Fumarsäure; Itaconsäure; Citraconsäure; Mesaconsäure; Aconitsäure; ihren Derivaten und Gemischen ausgewählt ist.

8. Zusammensetzung nach einem der vorhergehenden Ansprüche, **dadurch gekennzeichnet, dass** das Oligomer oder das von einem Polyolefin abgeleitete Polymer Polyisobutylen mit einer gegebenenfalls modifizierten, endständigen Bernsteinsäuregruppe ist.

9. Zusammensetzung nach einem der vorhergehenden Ansprüche, **dadurch gekennzeichnet, dass** das Oligomer oder das von einem Polyolefin abgeleitete Polymer bei der Reaktion von Maleinsäureanhydrid und Polyisobutylen gebildet wird.

10. Zusammensetzung nach einem der vorhergehenden Ansprüche, **dadurch gekennzeichnet, dass** die Menge des Oligomers/der Oligomere oder des von einem Polyolefin abgeleiteten Polymers /der von einem Polyolefin abgeleiteten Polymere im Bereich von 0, 1 bis 20 Gew.-% wirksame Substanz, bezogen auf das Gesamtgewicht der Zusammensetzung, liegt.

11. Zusammensetzung nach einem der vorhergehenden Ansprüche, wobei das wirksame desodorierende Salz unter den Antitranspirantien in Salzform ausgewählt ist,

12. Zusammensetzung nach Anspruch 11, wobei das Antitranspirantsalz unter den Salzen von Aluminium und/oder Zirconium sowie unter Komplexen von Zirconiumhydroxychlorid und von Aluminiumhydroxychlorid mit einer Aminosäure ausgewählt ist.

13. Zusammensetzung nach Anspruch 12, wobei das Antitranspirant ausgewählt ist unter Aluminumchlorohydrat in aktivierter oder nichtaktivierter Form. Aluminiumchlorohydrex, Aluminiumchlorohydrex-Polyethylenglycol-Komplex, Aluminiumchlorohydrex-Propylenglycol-Komplex, Aluminiumdichlorohydrat, A-luminiumdichlorohydrex-Polyethylenglycol-Komplex, Alumini-Umdichlorohydrex-Propylenglycol-Komplex, Aluminiumsesquichlorohydrat, Aluminiumesquichlorohydrex-Polyethylenglycol-Komplex, Aluminiumsesquichlorohydrex-Propylenglycol-Komplex, sowie mit Natriumlactat und Aluminiumlactat gepuffertem Aluminiumsulfat.

14. Zusammensetzung nach Anspruch 12, wobei das Antitranspirant unter Aluminium-Zirconium Octachlorohydrat, Aluminium-Zirconium Pentachlorohydrat, Aluminium-Zirconium Tetrachlorohydrat und Aluminium-Zirconium Trichlorohydrat ausgewählt ist.

15. Zusammensetzung nach Anspruch 12, wobei das Antitranspirant unter den Komplexen aus Zirkoniumhydroxychlorid und Aluminiumhydroxychlorid und Glycin (ZAG) ausgewählt ist.

16. Zusammensetzung nach Anspruch 15, wobei das Antitranspitant unter den Komplexen Aluminium-Zirconium Octachlorohydrex-Glycin, Aluminium-Zirconium Pentachlorohydrex-Glycin, Aluminium-Zirconium Tetrachlorohydrex-Glycin und Aluminium-Zirconium Trichlorohydrex-Glycin ausgewählt ist.

17. Zusammensetzung nach einem der Ansprüche 1 bis 10, wobei das wirksame desodorierende Salz unter bakteriostatischen oder bakteriziden Stoffen ausgewählt ist.

18. Zusammensetzung nach Anspruch 17, wobei das wirksame desodorierende Salz unter den quartären Ammononiumsalzen ausgewählt ist.

19. Zusammensetzung nach Anspruch 18, wobei das wirksame Salz unter den cetyltrimethylammoniumsalzen und Cetylpyridiniumsalzen ausgewählt ist.

20. Zusammensetzung nach einem der Ansprüche 1 bis 10, wobei das wirksame Salz ausgewählt ist unter
- Zinksalzen;
- Chlorhexidinsalzen;
- Polyhexamethylen-biguanidsalzen,
- Natriumbicarbonat.

21. Zusammensetzung nach Anspruch 20, wobei das Zinksalz unter Zinksalicylat, Zinkphenolsulfonat, Zinkpyrrolidoncarboxylat, Zinksulfat, Zinkchlorid, Zinklactat, Zinkgluconat, Zinkricinoleat, Zinkglycinat, Zinkcarbonat, Zinkcitrat, Zinklaurat, Zinkoleat, Zinkorthophosphat, Zinkstearat, Zinksilicat, Zinktartrat, oder Zinkacetat ausgewählt ist.

22. Zusammensetzung nach einem der Ansprüche 1 bis 21, wobei das wirksame desodorierende Salz wasserlöslich ist.

23. Zusammensetzung nach Anspruch 22, wobei das wasserlösliche, wirksame desodorierende Salz Aluminiumchlorohydrat in aktivierter oder nichtaktivierter Form ist.

24. Zusammensetzung nach einem der Ansprüche 1 bis 23, wobei das wirksame desodorierende Salz in einer Menge von 0, 1 bis 30 Gew.-%, bezogen auf die gesamte Zusammensetzung, und vorzugsweise in 10 bis 25 Gew.-% vorliegt.

25. Zusammensetzung nach einem der Ansprüche 1 bis 24, die ferner mindestens einen zusätzlichen, desodorierenden Wirkstoff enthält, der nicht in Form eines Salzes vorliegt.

26. Zusammensetzung nach Anspruch 25, wobei der zusätzliche desoderierende Wirkstoff unter 2,4,4'-Trichlor-2'-hydroxydiphenylether (Triclosan), 2,4-Dichlor-2'-hydroxydiphenylether, 3',4',5'-Trichlorsalicylanilid, 1-(3',4'-Dichlorphenyl)-3-(4'-chlorphenyl)harnstoff (Triclocarban) oder 3,7,11-Trimethyldodeca-2,5,10-trienol (Farnesol), Chlorhexidin, Diglycerolmonocaprat, Diglycerolmonolaurat oder Glycerolmonolaurat ausgewählt ist.

27. Zusammensetzung nach einem der Ansprüche 1 bis 26, **dadurch gekennzeichnet, dass** sie in einer mehr oder weniger verdickten Form, die mittels einer Tube oder eines Gitters verabreicht wird, oder in Form eines als Kugel geformten Roll-Ons, als Stift, Spray oder als Aerosol vorliegt.

28. Zusammensetzung nach einem der Ansprüche 1 bis 27, **dadurch gekennzeichnet, dass** die Ölphase ein oder mehrere, nicht siliconhaltige, flüchtige oder nichtflüchtige, weich machende Kohkenwasserstofföle enthält.

29. Zusammensetzung nach einem der Ansprüche 1 bis 28, **dadurch gekennzeichnet, dass** sie ferner mindestens ein Suspendiermittel enthält.

30. Zusammensetzung nach einem der Ansprüche 1 bis 30, **dadurch gekennzeichnet, dass** sie ferner mindestens ein organisches Pulver enthält.

31. Zusammensetzung nach einem der Ansprüche 1 bis 30, **dadurch gekennzeichnet, dass** sie des weiteren kosmetische Zusatzstoffe enthält, die unter Wachsen, beruhigenden Stoffen, Antioxidantien. Trübungsmitteln, Stabilisatoren, Hydratisierungsmitteln, Vitaminen, Parfums, Bakteriziden, Konservierungsmitteln, Polymeren, Parfums und Verdickungsmitteln ausgewählt sind.

32. Zusammnensetzung nach einem der Ansprüche 1 bis 31, **dadurch gekennzeichnet, dass** sie ferner ein oder mehrere strukturgebende Mittel oder Gelbildner der Ölphase aufweist.

33. Zusammensetzung nach einem der Ansprüche 1 bis 32, **dadurch gekennzeichnet, dass** sie in einem Aerosolbehälter unter Druck abgefüllt und dargereicht wird und dass sie ein oder mehrere Treibmittel enthält.

34. Kosmetisches Verfahren zur Behandlung der Transpiration, das im Auftragen einer wirksamen Menge einer in einem der Ansprüche 1 bis 33 definierten Zusammensetzung auf die Hautoberfläche besteht.

35. Kosmetiches Verfahren zur Behandlung von Körpergerüchen, die im Zusammenhang mit der Transpiration stechen, das im Auftragen einer wirksamen Menge einer Zusammensetzung, wie sie in einem der Ansprüche 1 bis 33 definiert ist, auf die Hautoberfläche besteht

36. Verwendung mindestens eines oligomeren Emulgators oder eines von einem Polyolefin abgeleiteten Polymers, das mindestens einen polaren Teil enthält, nach einem der Ansprüche 1 bis 9 um eine Wasser-in-Öl-Emulsion, die mindestens ein wirksames desodorierendes Salz enthält, zu stabilisieren.

37. Verwendung mindestens eines oligomeren Emulgators oder eines von einem Polyolefin abgeleiteten Polymers, das mindestens einen polaren Teil enthält, nach einem der Ansprüche 1 bis in einer in Form einer Wasser-in-Öl-Emulsion vorliegenden, kosmetischen Zusammensetzung, die mindestens ein wirksames desodorierendes Salz oder ein Antitranspirant enthält, um die Eigenschaften, die auf der Haut nach dem Auftragen dieser Zusammensetzung zu spüren send, zu verbessern.
